# EUROPÄISCHE PATENTANMELDUNG

(11) **EP 2 599 513 A1**
(43) Veröffentlichungstag der Anmeldung: **05.06.2013**
(21) Anmeldenummer: 11401649.6
(22) Anmeldetag: 01.12.2011
(51) Int. Cl.: A61M 15/06, A61M 11/04, A24F 47/00

(54) **Heißluftextraktionsinhalator mit Temperaturschalter**

(71) Anmelder: Stobi GmbH & Co. KG, 78532 Tuttlingen (DE)
(72) Erfinder: Storz, Markus, 78532 Tuttlingen (DE)
(74) Vertreter: Späth, Dieter

(57) **Zusammenfassung**

Die Erfindung betrifft einen Heißluftextraktionsinhalator (1) mit einem beheizten Wärmetauscher (3) zur Erzeugung eines heißen Luftstroms und mit einem Temperaturschalter (10), der auf eine Heizeinrichtung (7) des Wärmetauschers (3) Temperatur abhängig schaltend oder regelnd einwirkt. Erfindungsgemäß ist vorgesehen, dass der Temperaturschalter (10), der vorzugsweise als Bimetallregler ausgebildet ist, wahlweise von einem Temperatur abhängigen stabilen Einschaltzustand in einen Temperatur abhängigen instabilen Regelzustand oder von einem Temperatur abhängigen stabilen Einschaltzustand in einen Temperatur unabhängigen stabilen Ausschaltzustand umschaltbar ist.

## Beschreibung

Die Erfindung betrifft einen Heißluftextraktionsinhalator, mit einem beheizten Wärmetauscher zur Erzeugung eines heißen Luftstroms und mit einem Temperaturschalter, der auf eine Heizeinrichtung des Wärmetauschers temperaturabhängig schaltend oder regelnd einwirkt.

Heißluftextraktionsinhalatoren dienen zur thermischen Verdampfung und anschließenden Inhalation von verdampfbaren, also unter Wärmeeinwirkung Aerosol bildenden Substanzen. Diese Substanzen werden mit Heißluft durch strömt, wodurch die Substanzen verdampfen bzw. ausdünsten und in die Heißluft übergehen. Unter Heißluft soll hier Luft verstanden werden, die typisch auf etwa 200 °C oder mehr erwärmt ist. Eine niedrigere Lufttemperatur ist ebenfalls möglich, sofern sie zum Verdampfen der in den Substanzen enthaltenen Aromen und Wirkstoffen ausreicht. Diese zum Beispiel mit Aromen und/oder Wirkstoffen versetzte Luft wird, nachdem sie auf eine zum Einatmen angenehme Temperatur herunter gekühlt ist, inhaliert, wobei die Wirkstoffe in der Einatemluft über die Lunge in den Blutkreislauf gelangen. Zum Stand der Technik wird beispielhaft auf die Druckschriften DE 198 03 376 C1 und DE 100 42 396 B4 verwiesen.

Bekannte Heißluftextraktionsinhalatoren weisen beispielsweise als Wärmetauscher einen massiven Aluminiumblock auf, der mit wenigstens einem Luftkanal durchzogen ist. Der Aluminiumblock ist üblicherweise gas- oder elektrobeheizt. Für eine gleichbleibende Temperatur der Einatemluft und um eine Überhitzung der in einer Füllkammer des Heißluftextraktionsinhalators aufgenommenen beim Erwärmen Aerosol bildenden Substanzen zu verhindern, wird die Temperatur des Luftstroms üblicherweise mittels einer Temperaturregelung in etwa konstant gehalten. Dabei genügt es meist, den Wärmetauscher so zu dimensionieren, dass er einen bestimmten maximalen Luftvolumenstrom auf die gewünschte Temperatur bringt und die Temperatur des Wärmetauschers zu regeln.

Bei elektrisch beheizten Wärmetauschern ist dazu in der Regel ein einstellbarer Bimetall-Thermostat vorgesehen, der die Spannungsversorgung zu einer elektrischen Heizeinrichtung des Wärmetauschers temperaturabhängig ein- und ausschaltet. Fällt die Temperatur des Wärmetauschers ab, so reagiert die Temperaturregelung darauf mehr oder weniger zeitverzögert in Abhängigkeit von einer Hysterese des Bimetalls. Ein solcher Temperaturschalter hält den Wärmetauscher über einen beliebig langen Zeitraum auf annähernd konstanter Temperatur. Er ist jedoch nicht geeignet, um die Heizeinrichtung des Wärmetauschers zum Vorheizen des Wärmetauschers einmal einzuschalten, bis der Wärmetauscher die gewählte Temperatur erreicht hat oder um die Heizung endgültig auszuschalten, wenn der Inhalationsvorgang beendet ist.

Der Erfindung liegt daher die Aufgabe zu Grunde, einen Heißluftextraktionsinhalator mit einem Temperaturschalter vorzuschlagen, bei dem der Temperaturschalter gegenüber dem Stand der Technik zusätzliche Schaltaufgaben wahrnimmt.

Diese Aufgabe wird erfindungsgemäß durch einen Heißluftextraktionsinhalator mit den Merkmalen des Anspruchs 1 gelöst. Weitere vorteilhafte Ausgestaltungen sind den Unteransprüchen zu entnehmen.

Bei dem erfindungsgemäßen Heißluftextraktionsinhalator weist der Temperaturschalter für die Heizung des Wärmetauschers einen Temperatur abhängigen stabilen Einschaltzustand (Das erste Aufheizen bis zur eingestellten Temperatur nach dem ersten Einschalten des Inhalators oder das erste Aufheizen nach Betätigen der manuellen Rücksetzeinrichtung), einen Temperatur abhängigen instabilen Regelzustand (Das fortwährende Einregeln der eingestellten Temperatur) und einen Temperatur unabhängigen stabilen Ausschaltzustand (Abkühlen bis Umgebungstemperatur) auf. Er schaltet wahlweise von dem Temperatur abhängigen stabilen Einschaltzustand in den Temperatur abhängigen instabilen Regelzustand oder von dem Temperatur abhängigen stabilen Einschaltzustand in den Temperatur unabhängigen stabilen Ausschaltzustand. In beiden Fällen ist der Einschaltzustand so lange stabil, bis eine vorzugsweise einstellbare Soll-Temperatur des Wärmetauschers erreicht ist. Beim Erreichen der Soll-Temperatur schaltet der Temperaturschalter die Heizung des Wärmetauschers aus oder verringert ihre Heizleistung. Für den Normalbetrieb des Heißluftextraktionsinhalators, insbesondere während des Inhalierens, schaltet der Temperaturschalter derart um, dass er von dem Temperatur abhängigen stabilen Einschaltzustand in den Temperatur abhängigen instabilen Regelzustand schaltet.

Für andere Betriebsfälle des erfindungsgemäßen Heißluftextraktionsinhalators kann der Temperaturschalter vorzugsweise manuell umgeschaltet werden, so dass der Temperaturschalter von dem bis zum Erreichen der Soll-Temperatur des Wärmetauschers stabilen Temperatur abhängigen Einschaltzustand in den Temperatur unabhängigen stabilen Ausschaltzustand schaltet, sobald der Wärmetauscher auf die Soll-Temperatur aufgeheizt ist. Im stabilen Temperatur unabhängigen Ausschaltzustand verbleibt der Temperaturschalter auch wenn die Temperatur des Wärmetauschers gegenüber der Soll-Temperatur bis auf die Raumtemperatur abfällt. Der Temperaturschalter kann beispielsweise ein elektromechanischer oder ein elektronischer Schalter sein oder elektromechanische und elektronische Schaltelemente oder Bauteile in Kombination aufweisen.

Vorzugsweise weist der Temperaturschalter eine manuelle Rücksetzeinrichtung für ein Umschalten aus dem stabilen Temperatur unabhängigen Ausschaltzustand in den Temperatur abhängig stabilen Einschaltzustand auf. Ohne bewusste manuelle Einwirkung auf die Rücksetzeinrichtung verbleibt der Temperaturschalter dauerhaft in dem stabilen Temperatur unabhängigen Ausschaltzustand. Dies verhindert zuverlässig, dass der erfindungsgemäße Heißluftextraktionsinhalator unbeabsichtigt dauerhaft in Betrieb bleibt. Der Temperaturschalter wirkt somit als Sicherheitseinrichtung für den Heißluftextraktionsinhalator, der ein Überhitzen verhindert. Er verhindert nicht nur eine andauernde Beheizung des Inhalators, sondern dadurch auch ein Überhitzen und eventuelles Anbrennen der zu verdampfenden Substanzen. Zusätzlich verringert ein derartiger Temperaturschalter die Brandgefahr für die Umgebung, in der Regel Wohnräume. Ein zusätzlicher Vorteil des erfindungsgemäßen Temperaturschalters ist die Möglichkeit, den Wärmetauscher einmal auf die Solltemperatur aufzuheizen, indem er manuell mit der Rücksetzeinrichtung in den temperaturabhängigen stabilen Einschaltzustand geschaltet wird. Nach Erreichen der Solltemperatur schaltet der Temperaturschalter bleibend aus, wenn nicht die Rücksetzeinrichtung betätigt wird.

Vorzugsweise ist der Temperaturschalter ein einstellbarer Thermostat. Dies ermöglicht die Soll-Temperatur des Wärmetauschers beliebig variabel einzustellen und damit die in dem Wärmetauscher gespeicherte Wärme zu variieren und insbesondere an Quantität und Qualität der in die Füllkammer verbrachten Substanzen anzupassen, um einen optimale Wirkstoffgehalt in der Inhalationsluft zu erreichen.

In einer bevorzugten Ausführungsform der Erfindung ist der Temperaturschalter ein Bimetallregler mit einem Schnappkontakt, wobei der Bimetallregler als Rücksetzeinrichtung vorzugsweise ein Betätigungselement aufweist, das stellungsabhängig den Temperatur abhängigen instabilen Regelzustand oder den Temperatur unabhängigen stabilen Ausschaltzustand des Temperaturschalters bestimmt.

So öffnet der Schnappkontakt momentartig bei Erreichen der eingestellten Temperatur. Die Wiedereinschaltung wird nach Absinken der Temperatur durch

Eindrücken des Betätigungselementes vorgenommen. Bei eingedrücktem Betätigungselement arbeitet der Temperaturschalter wie ein Temperaturregler.

Bimetallregler sind in vielen Ausführungsformen bekannt. Sie werden kostengünstig angeboten und weisen außerdem eine lange Lebensdauer auf, in der sie zuverlässig schalten. Ihre Größe und Bauform kann vom Hersteller auf einfache Weise an die Bedürfnisse der Anwendung angepasst werden. Dies gilt auch für die Form des Betätigungselementes, das üblicherweise aus einem elektrisch isolierenden Material hergestellt ist. Das Betätigungselement kann dabei so ausgebildet sein, dass es per Hand direkt oder indirekt über ein Bedienelement betätigbar ist.

Vorteilhafterweise stützt das Betätigungselement den Schnappkontakt für den Temperatur abhängig instabilen Regelzustand vor einem Schnapppunkt ab. Beim Verlassen des stabilen Einschaltzustandes unter Einwirkung des Bimetalls des Temperaturschalters verhindert das Betätigungselement, dass sich der Schnappkontakt in den vorgesehenen stabilen Ausschaltzustand bewegt. Es legt den instabilen Regelzustand des Temperaturschalters fest, d. h. diejenige Position, die der Schnappkontakt in einer Endstellung beim Schalten einnehmen kann.

Bei einer Variante des erfindungsgemäßen Heißluftextraktionsinhalators ist die Stellung des Betätigungselementes bezüglich dem Schnapppunkt mit einem Bedienelement veränderbar. Das Bedienelement ist dabei vorzugsweise so ausgebildet, dass es in einer bestimmten Stützstellung das Betätigungselement vor dem Schnapppunkt des Schnappkontaktes abstützt, wobei die Stützstellung durch einen Anschlag für das Bedienelement genau bestimmt ist. Vorzugsweise ist das Bedienelement selbstständig rückstellend ausgebildet, so dass es bei Nichtbetätigung automatisch eine Grundstellung einnimmt, in der es nicht auf das Betätigungselement einwirkt. Ohne Einwirkung des Bedienelements auf die Rücksetzeinrichtung des Temperaturschalters, insbesondere auf das Betätigungselement der Rücksetzeinrichtung schaltet der Temperaturschalter, sobald die für den Wärmetauscher vorgewählte Temperatur erreicht ist, von dem Temperatur abhängigen stabilen Einschaltzustand in den stabilen und damit Temperatur unabhängigen Ausschaltzustand.

Bei einer bevorzugten Ausführungsform der Erfindung ist der Heißluftextraktionsinhalator als Handgerät ausgeführt und weist einen seitlich von einem Inhalatorgehäuse abstehenden Haltegriff auf. Mittels dem Haltegriff kann der Heißluftextraktionsinhalator während des Inhalierens bequem auf Brusthöhe gehalten werden. Weiterer Vorteil ist, dass der Wärmetauscher entfernt von dem Haltegriff angeordnet ist und sich damit beim Betrieb des Heißluftextraktionsinhalator nur gering erwärmt. Günstigerweise ist das Bedienelement für das Betätigungselement des Temperaturschalters nach Art eines Abzugs oder eines Scherengriffes ausgebildet und an dem Haltegriff angeordnet. So kann auf einfache Weise mit einer Hand der Heißluftextraktionsinhalator am Haltegriff gehalten und gleichzeitig das Bedienelement betätigt werden. Da das Bedienelement selbstständig rückstellend ausgebildet ist, löst es sich beim Abstellen oder Ablegen des Heißluftextraktionsinhalators automatisch von dem Betätigungselement, so dass der Temperaturschalter nach Erreichen der Solltemperatur bleibend ausschaltet, d.h. in den Temperatur unabhängigen stabilen Ausschaltzustand schaltet. Auch eine erneute Inbetriebnahme des Heißluftextraktionsinhalators ist einfach, da der Temperaturschalter durch Betätigen des Bedienelementes ohne weiteres Zutun wieder in den Temperatur abhängigen stabilen Einschaltzustand schaltet.

Nachfolgend wird die Erfindung anhand eines in der Zeichnung dargestellten Ausführungsbeispiels näher erläutert. Weitere Merkmale der Erfindung ergeben sich aus der folgenden Beschreibung des Ausführungsbeispiels der Erfindung in Verbindung mit den Ansprüchen und der beigefügten Zeichnung. Die einzelnen Merkmale der Erfindung können für sich allein oder zu mehreren bei unterschiedlichen Ausführungsformen der Erfindung verwirklicht sein. Es zeigen:
- Figur 1: einen erfindungsgemäßen Heißluftextraktionsinhalator in Seitenansicht;

- Figur 2: den Heißluftextraktionsinhalator gemäß Figur 1 in einer Achsschnittdarstellung; und
- Figur 3: den Temperaturschalter aus Figur 2 in einer Ausschnittsvergrößerung.

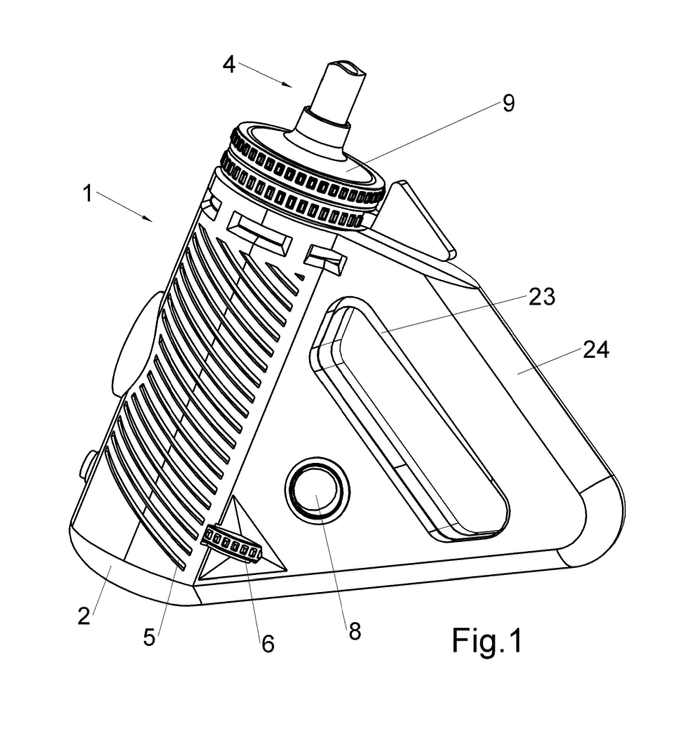

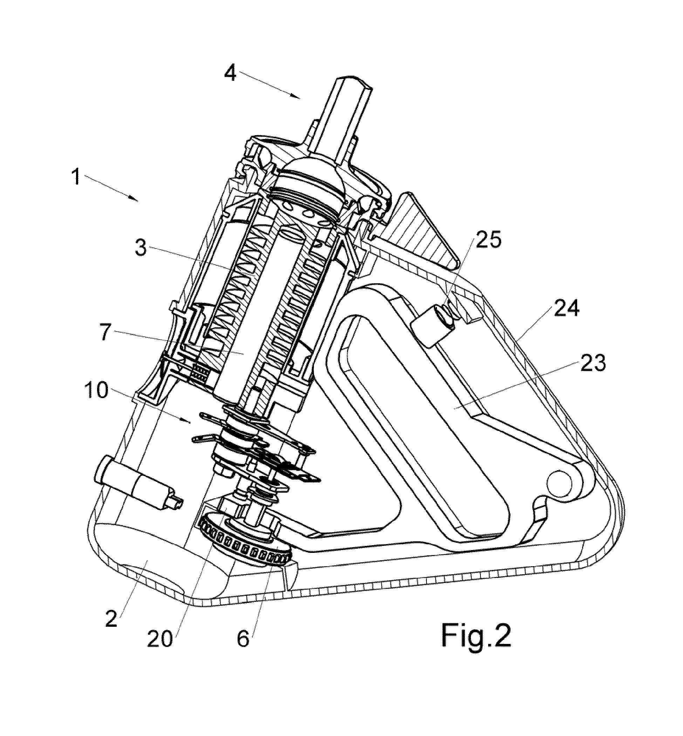

Die Figur 1 zeigt ein Ausführungsbeispiel des erfindungsgemäßen Heißluftextraktionsinhalators 1 mit einem Inhalatorgehäuse 2, in dem ein zu dem Inhalatorgehäuse 2 hin wärmeisolierter Wärmetauscher 3 zum Erwärmen eines Luftstroms angeordnet ist, der bei Beaufschlagung eines Inhalatorauslasses 4 des Heißluftextraktionsinhalators 1 mit Unterdruck an Belüftungsschlitzen 5 in das Inhalatorgehäuse 2 eintritt und nach dem Passieren des Wärmetauschers 3 an dem Inhalatorauslass 4 austritt. Der Wärmetauscher 3 ist elektrisch beheizt, wobei dessen Temperatur mit einem Temperaturwähler 6 einstellbar ist und eine Versorgungsspannung einer in der Figur 2 erkennbaren elektrischen Heizpatrone 7 über einen Netzspannungsschalter 8 schaltbar ist.

Figur 2 zeigt das Innere des Inhalatorgehäuses 2 in einer Achsschnittdarstellung. Als wesentliche Teile weist das Inhalatorgehäuse 2 den Wärmetauscher 3 und eine Füllkammer 9 für unter Hitzeeinwirkung Aerosol bildende Substanzen auf, die in Verlängerung des Wärmetauschers 3 zwischen dem Inhalatorauslass 4 und dem Wärmetauscher 3 angeordnet ist. Mit der Heizpatrone 7 und dem Netzschalter 8 ist in Reihe geschaltet ein Temperaturschalter 10 in Form eines Bimetallreglers 10, der die Temperatur des Wärmetauschers 3 regelt, indem er Temperatur abhängig die Versorgungsspannung der elektrischen Heizpatrone 7 ein- und ausschaltet.

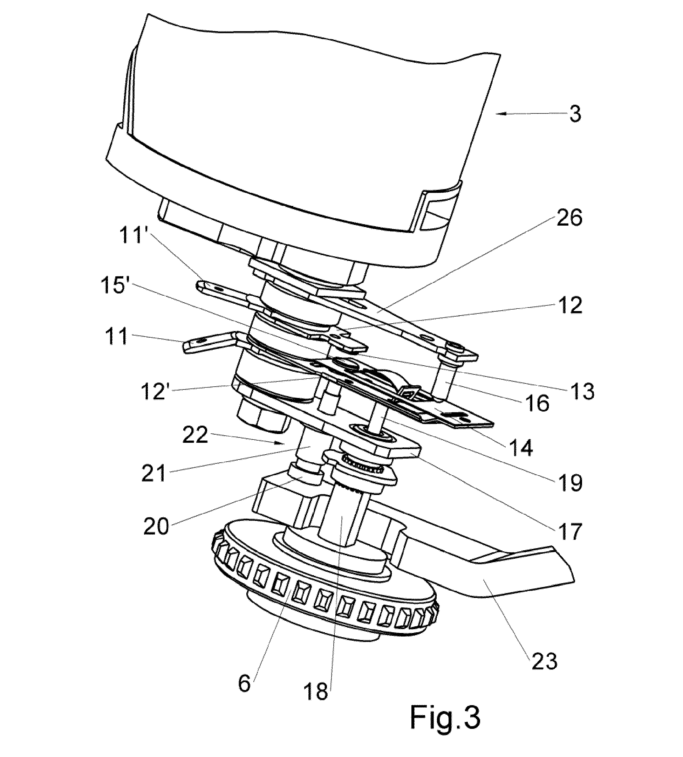

Die Figur 3 zeigt eine Ausschnittsvergrößerung des Bimetallreglers 10, der voneinander elektrisch isolierte Anschlüsse 11, 11' zum Anschluss von in der Figur 3 nicht dargestellten elektrischen Verbindungsleitungen aufweist, die mit Kontaktstreifen 12, 12' des Bimetallreglers 10 elektrisch leitend verbunden sind. Der erste Kontaktstreifen 12 ist unbeweglich ausgebildet und trägt einen ersten ruhenden Schaltkontakt 13. Der zweite Kontaktstreifen 12' ist biegeelastisch und trägt einen federnd ausgebildeten Sprungkontakt 15, der mit dem Kontaktstreifen 12' elektrisch verbunden ist und mittels des Sprungschalters 14 abhängig von der Stellung des Vorspannstiftes 19 und der Stellung des Übertragungsstiftes 16 den Kontakt schließt oder öffnet.

Der Bimetallstreifen 26 krümmt sich bei zunehmender Temperatur in Richtung Sprungschalter 14. Die Krümmung wird vom Übertragungsstift 27 auf den Sprungschalter 14 übertragen, welcher dann abhängig von der Stellung des Vorspannstiftes 19 bei Erreichen der Solltemperatur den Sprungkontakt 15 vom ruhenden Schaltkontakt 13 wegspringen lässt und so die Stromzufuhr zur Heizpatrone 7 unterbricht.

Bei abnehmender Temperatur geht der Bimetallstreifen 26 mitsamt dem Übertragungsstift 16 wieder in Richtung seiner Ausgangsposition weg vom Sprungschalter 14, wodurch sich dieser wieder entspannt und abhängig von der Stellung des Vorspannstiftes 19 den Sprungkontakt 15 wieder zum ruhenden Schaltkontakt 13 hinspringen lässt. Die Stromzufuhr zur Heizpatrone 7 wird so wieder hergestellt und damit der Wärmetauscher wieder aufgeheizt. Die zunehmende Temperatur bewirkt dann wieder ein Krümmen des Bimetallstreifens 26 mit den zuvor beschrieben Folgen. Dieser Temperatur abhängige instabile Regelzustand wird solange wiederholt, bis ein Temperatur unabhängiger stabiler Ausschaltzustand herbeigeführt wird.

Ein Schaltpunkt des Bimetallreglers 10 kann mittels des Temperaturwählers 6 eingestellt werden, der auf einer von einem Haltestreifen 17 getragenen Drehwelle 18 angeordnet ist. Die Drehwelle 18 ist in axialer Richtung gegenüber dem Haltestreifen 17 mittels an dem Haltestreifen 17 und der Drehwelle 18 vorgesehenen, in der Zeichnung nicht sichtbaren Gewinden bewegbar.

Der Haltestreifen 17 und Sprungschalter 14 erstrecken sich im Wesentlichen parallel zueinander, so dass ein Vorspannstift 19 der Drehwelle 18, der sich an dem Sprungschalter 14 in etwa mittig abstützt, senkrecht auf den Sprungschalter 14 einwirken kann. Durch Drehen der Drehwelle 18 wird für die Schalttemperatureinstellung des Temperaturschalters 10 der elastisch verformbare Sprungschalter 14 mehr oder weniger vorgespannt, so dass der Sprungkontakt 15 bei Temperaturerhöhung des Temperaturschalters 10 abhängig von der Drehstellung der Drehwelle 18 früher oder später von dem ruhenden Schaltkontakt 13 des Kontaktstreifens 12 wegspringt.

Beim Temperatur abhängig stabilen Einschaltzustand des Bimetallreglers 10 liegen der Schaltkontakt 13 und der Sprungkontakt 15 aneinander an und werden durch den Druck des Sprungschalters 14 gegeneinander gedrückt. Nach dem Erreichen der eingestellten Solltemperatur springt der Sprungkontakt 15 vom ruhenden Schaltkontakt 13 weg, wobei sich der Sprungschalter 14 wahlweise in einen instabilen Regelzustand oder in einen stabilen Ausschaltzustand bewegt. Aus dem instabilen Regelzustand bringt der Sprungschalter 14 bei sinkender Temperatur den Schaltkontakt 13 und den Sprungkontakt 15 automatisch wieder zusammen, während aus dem stabilen Ausschaltzustand heraus dies manuell erfolgen muss. Dazu ist an dem Haltestreifen 17 ein beweglich geführtes Betätigungselement 20 angeordnet, mit dem der Sprungkontakt 15 in Richtung des ruhenden Schaltkontaktes 13 bewegbar ist. Das Betätigungselement 20 ist mit dem Sprungkontakt 15 des Sprungschalters 14 fluchtend vorgesehen und bildet mit einer zugeordneten Führung 21 die Rücksetzeinrichtung 22 des Temperaturschalters 10 des Heißluftextraktionsinhalators 1.

Das Betätigungselement 20 bestimmt stellungsabhängig den instabilen Regelzustand oder den stabilen Ausschaltzustand des Bimetallreglers 10. Für den instabilen Regelzustand stützt das Betätigungselement 20 den Sprungkontakt 15 vor einem Schnapppunkt ab. Dazu wird es manuell in eine bestimmte Position vor dem Schnapppunkt gegenüber dem Haltestreifen 17 gebracht und dort manuell gehalten. Für ein bequemes und einfaches Bedienen des Betätigungselementes 20 ist ein Bedienelement 23 vorgesehen, mit dem die Stellung des Betätigungselementes 20 bezüglich dem Schnapppunkt veränderbar ist. Wie der Figur 2 entnehmbar, ist das Bedienelement 23 selbstständig rückstellend ausgebildet und stützt das Betätigungselement 20 bei Betätigung axial ab. Dies ist besonders dann von Vorteil, wenn der Heißluftextraktionsinhalator 1 als Handgerät ausgebildet ist. Beim Abstellen oder Ablegen eines solchen Heißluftextraktionsinhalators 1 wirkt das Bedienelement 23 nicht mehr auf das Betätigungselement 20 abstützend ein, wodurch der Temperaturschalter 10 automatisch in den stabilen Temperatur unabhängigen Ausschaltzustand umschaltet.

Wie aus den Figuren 1 und 2 ersichtlich ist der Heißluftextraktionsinhalator 1 als Handgerät ausgeführt und weist einen seitlich von dem Inhalatorgehäuse 2 abstehenden Haltegriff 24 auf, der entfernt von dem Wärmetauscher 3 angeordnet ist. Das Bedienelement 23 für das Betätigungselement 20 des Temperaturschalters 10 ist nach Art eines Abzugs oder eines Scherengriffes ausgebildet und an dem Haltegriff 24 angeordnet. Dadurch ist das Bedienelement 23 durch Umgreifen des Haltegriffs 24 betätigbar. Das Bedienelement 23 ist selbstständig rückstellend durch eine in der Figur 2 sichtbare sich an dem Haltegriff 24 und dem Bedienelement 23 abstützende Schraubenfeder 25, es löst sich beim Loslassen des Bedienelementes 23 automatisch von dem Betätigungselement 20, so dass der Temperaturschalter 1 nach Erreichen der Soll-Temperatur in den Temperatur unabhängigen stabilen Ausschaltzustand schaltet. Wird der Haltegriff 24 umgriffen und dabei das Bedienelement 23 betätigt, schaltet der Temperaturschalter 1 bei Erreichen der Soll-Temperatur in den Temperatur abhängigen instabilen Regelzustand.

## Patentansprüche

1. Heißluftextraktionsinhalator (1) mit einem beheizten Wärmetauscher (3) zur Erzeugung eines heißen Luftstroms und mit einem Temperaturschalter (10), der auf eine Heizeinrichtung (7) des Wärmetauschers (3) Temperatur abhängig schaltend oder regelnd einwirkt, **dadurch gekennzeichnet, dass** der Temperaturschalter (10) einen Temperatur abhängigen stabilen Einschaltzustand, einen Temperatur abhängigen instabilen Regelzustand und einen Temperatur unabhängigen stabilen Ausschaltzustand aufweist, und dass der Temperaturschalter (10) von dem Temperatur abhängigen stabilen Einschaltzustand wahlweise in den Temperatur abhängigen instabilen Regelzustand oder in den Temperatur unabhängigen stabilen Ausschaltzustand umschaltet.

2. Heißluftextraktionsinhalator nach Anspruch 1, **dadurch gekennzeichnet, dass** der Temperaturschalter (10) eine manuelle Rücksetzeinrichtung (22) für eine Rücksetzung von dem stabilen Ausschaltzustand in den stabilen Einschaltzustand aufweist.

3. Heißluftextraktionsinhalator nach Anspruch 1 oder 2, **dadurch gekennzeichnet, dass** der Temperaturschalter (10) ein einstellbarer Thermostat ist.

4. Heißluftextraktionsinhalator nach Anspruch 3, **dadurch gekennzeichnet, dass** der Thermostat ein Bimetallregler (10) mit einem Sprungkontakt (14) ist.

5. Heißluftextraktionsinhalator nach Anspruch 4, **dadurch gekennzeichnet, dass** der Bimetallregler (10) als Rücksetzeinrichtung (22) ein Betätigungselement (20) aufweist, das stellungsabhängig den instabilen Regelzustand oder den stabilen Ausschaltzustand des Temperaturschalters (10) bestimmt.

6. Heißluftextraktionsinhalator nach Anspruch 5, **dadurch gekennzeichnet, dass** das Betätigungselement (20) den Sprungkontakt (14) wahlweise vor einem Schnapppunkt abstützt.

7. Heißluftextraktionsinhalator nach Anspruch 5 oder 6, **dadurch gekennzeichnet, dass** mit einem Bedienelement (23) des Heißluftextraktionsinhalators (1) die Stellung des Betätigungselementes (20) bezüglich dem Schnapppunkt veränderbar ist.

8. Heißluftextraktionsinhalator nach Anspruch 7, **dadurch gekennzeichnet, dass** das Bedienelement (23) beim Betätigen das Betätigungselement (20) abstützt und selbstständig rückstellend ausgebildet ist.

9. Heißluftextraktionsinhalator nach einem der vorangegangen Ansprüche, **dadurch gekennzeichnet, dass** der Heißluftextraktionsinhalator (1) als Handgerät ausgeführt ist und einen seitlich an einem Inhalatorgehäuse (2) angeordneten Haltegriff (24) aufweist.

10. Heißluftextraktionsinhalator nach Anspruch 9, **dadurch gekennzeichnet, dass** das Bedienelement (23) am Haltegriff (24) angeordnet und nach Art eines Abzugs oder eines Scherengriffes ausgebildet ist.
